# EUROPÄISCHE PATENTANMELDUNG

(11) **EP 0 521 264 A2**
(43) Veröffentlichungstag der Anmeldung: **07.01.1993**
(21) Anmeldenummer: 92107898.6
(22) Anmeldetag: 11.05.1992
(51) Int. Cl.: H01Q 9/30, A61F 7/12, A61N 5/04

(54) **Antennenanordnung mit Zuleitung**

(30) Priorität: 03.07.1991 DE 4122050
(71) Anmelder: W.L. Gore & Associates GmbH, D-85636 Putzbrunn (DE)
(72) Erfinder: Gronauer, Volker, W-8832 Weissenburg (DE)
(74) Vertreter: Klunker . Schmitt-Nilson . Hirsch

(57) **Zusammenfassung**

Eine Antennenanordnung mit Zuleitung in Form einer hochfrequenztauglichen Koaxialkabelanordnung, insbesondere zur medizinischen Wärmeapplikation in Körperhohlräumen, ein Extrudierverfahren zu ihrer Herstellung, ein Dilatationskatheter mit der Antennenanordnung sowie ein Verfahren zur Ausführung einer Dilatationsbehandlung werden offenbart. Die Koaxialkabelanordnung mit einem durchgehenden Innenleiter (1), einem zylindrisch um den Innenleiter (1) angeordneten Isolator (2), einer in einem am proximalen Ende (5) der Koaxialkabelanordnung beginnenden ersten Abschnitt (A) der Koaxialkabelanordnung auf den Isolator (2) aufgebrachten Abschirmeinrichtung (3), einem am distalen Ende (10) des ersten Abschnittes (A) der Koaxialkabelanordnung beginnenden und am distalen Ende (6) der Koaxialkabelanordnung endenden zweiten Abschnitt (B) der Koaxialkabelanordnung als Antenne (8), in dem kein Isolator (2) und keine Abschirmeinrichtung (3) um den durchgehenden Innenleiter (1) herum angeordnet sind, wobei die Koaxialkabelanordnung einen dritten Abschnitt (C) aufweist, der am proximalen Ende (5) der Antennenanordnung beginnt und der kürzer ist als der erste Abschnitt (A) der Koaxialkabelanordnung, weist in dem dritten Abschnitt (C) beispielsweise durch Verfüllen von der Abschirmeinrichtung inhärenten Wirk-, Flecht- oder Wickellücken eine höhere Steifigkeit auf.

## Beschreibung

Die Erfindung betrifft eine stabförmige Antennenanordnung mit Zuleitung in Form einer hochfrequenztauglichen Koaxialkabelanordnung, insbesondere zur medizinischen Wärmeapplikation in Körperhohlräumen, wie sie beispielsweise in speziellen Ballonkathetern zur Koronarangioplastie eingesetzt werden kann.

Nach einer Beseitigung von Durchblutungsstörungen mittels Aufweiten oder Sprengen von durch Ablagerungen (atheronatös) bedingten Verengungen beispielsweise in Becken-, Bein- oder Koronararterien durch perkutane transluminale Rekanalisierung, im folgenden als "Dilatation" bezeichnet, tritt häufig eine arterielle Dissektion, d.h. eine durch ärztlichen Eingriff (iatrogen) verursachte Verletzung der Arterienwand, auf. Hautlappen, die von der Gefäßwand in das Gefäß hineinhängen, hemmen den Blutfluß und können zum Gefäßverschluß oder sogar - bei Koronararterien - zum Infarkt führen. Zur Vermeidung dieser Komplikation bieten sich zwei Alternativen an: Zum einen können mittels eines Ballonkatheters plazierte Drahtgeflechte ("Stents") implantiert werden, die die Gefäßwand mechanisch stabilisieren und so das Gefäß offenhalten. Dieses Vorgehen weist den Nachteil einer großen Gefahr der Thrombosenbildung auf. Zum anderen ist aus Rosen, A. et al.: "Percutaneous Transluminal Microwave Balloon Angioplasty", Transactions on Microwave Therory and Techniques, Band 38, N^{º} 1, Januar 1990, ein Verfahren bekannt, bei dem ein mit einem zur Abstrahlung von elektromagnetischen Wellen im Mikrowellenbereich geeigneten asymmetrischen Dipol versehener und in der Regel mehrlumiger Ballonkatheter ("Hot Balloon") eingesetzt wird, der durch ein in ein Lumen eingelegtes Kabel mit Hochfrequenzenergie gespeist wird. Dieser Dilatationskatheter wird in drucklosem Zustand und ohne Zufuhr von Hochfrequenzenergie in das betreffende Gefäß eingeführt und mit Druck beaufschlagt, so daß die gewünschte Aufweitungs- oder Sprengwirkung erzielt wird. Daran anschließend wird dem im Katheter angebrachten Dipol Mikrowellen-Hochfrequenzenergie zugeführt, so daß die Gefäßwand und die übrige unmittelbare Umgebung des Katheterballons kurzzeitig auf eine Temperatur von beispielsweise mehr als 50°C erwärmt werden. Dadurch werden Eiweißsubstanzen zum Gerinnen gebracht, um Hautlappen mit der Gefäßwand zu verkleben.

Durch die in einen Dilatationskatheter eingesetzte Koaxialkabelanordnung muß einerseits die zur Gewebeerwärmung dienende Mikrowellen-Hochfrequenzenergie bei möglichst geringen Verlusten zum Antennendipol im Ballon geleitet werden. Weist das verwendete Koaxialkabel eine zu hohe Dämpfung auf, so ist dies zwangsläufig mit einer unerwünschten Wärmeerzeugung in dem Lumen des Katheters verbunden, in den die Koaxialkabelanordnung eingesetzt ist. Diese unerwünschte Wärmeerzeugung kann beispielsweise zu einer gefährlichen Koagulation des den Katheterschlauch umgebenden Blutes führen. Andererseits muß die Kabelanordnung im distalen Bereich mit geringen Kräften leicht biegbar sein, damit sie insbesondere im Koronarbereich auch durch enge Biegungen hindurch in das betreffende Gefäß eingeführt werden kann, während im proximalen Bereich eine Mindeststeifigkeit der Kabelanordnung erforderlich ist, um sie in enge Lumen eines Katheters einführen und sodann darin vorschieben zu können. Darüber hinaus sollte die Kabelanordnung einen Durchmesser aufweisen, der so gering wie möglich ist, damit der Lumen ebenfalls mit geringem Querschnitt ausgeführt werden kann.

Daher werden in Dilatationskathetern bislang Koaxialkabel eingesetzt, die als Schirm beispielsweise einen gewickelten oder geflochtenen Flachbandleiter oder Rundleiter aufweisen, der im proximalen Bereich mit einem dünnen Metallröhrchen überzogen ist, um dort die Steifigkeit zu erhöhen. Dieser Aufbau weist jedoch den Nachteil auf, daß ein Metallröhrchen, das auf ein Kabel aufgeschoben werden soll, infolge der dafür benötigten Festigkeit eine verhältnismäßig große Wandstärke aufweisen muß und führt somit zu einem unerwünschten Anwachsen des effektiven Kabeldurchmessers. Dadurch verringert sich der im Lumen zur Verfügung stehende Spielraum zum Verschieben des Kabels; unter Umständen kann die Wahl eines Katheterschlauches mit größerem Durchmesser erforderlich werden. Auch ist die Fertigung einer derartigen Kabelanordnung aufwendig und unter wirtschaftlichen Gesichtspunkten nachteilig, da das aufgeschobene Metallröhrchen in einem gesonderten Fertigungsschritt auf geeignete Weise mit dem Koaxialkabel verbunden werden muß.

Ein anderer Ansatz, der für Kabelanordnungen in Dilatationskathetern verwendet wird, besteht darin, zwei Kabelsektionen unterschiedlicher Steifigkeit getrennt zu fertigen und an einer gemeinsamen Schnittstelle mit einer Mikrosteckverbindung elektrisch und mechanisch miteinander zu verbinden. Abgesehen von der Verschlechterung der elektrischen Übertragungseigenschaften der Kabelanordnung durch die zusätzliche durch die Schnittstelle bedingte Dämpfung und dem hohen konstruktiven und fertigungstechnischen Aufwand hierfür muß dieser Ansatz auch deswegen als nachteilig angesehen werden, weil für den Patienten das zusätzliche Risiko eines fehlerhaften Öffnens der Verbindung während eines Eingriffes besteht.

Aufgabe der Erfindung ist es daher, eine verbesserte Antennenanordnung mit Zuleitung in Form einer hochfrequenztauglichen Koaxialkabelanordnung insbesondere zur medizinischen Wärmeapplikation in Körperhohlräumen nach dem Oberbegriff der Ansprüche 1, 4 oder 5 verfügbar zu machen, die einfach und wirtschaftlich zu fertigen ist, die hervorragende elektrische Übertragungseigenschaften sowie einen geringen Durchmesser aufweist und die bei der Anwendung einfach, schnell und sicher gehandhabt werden kann.

Diese Aufgabe wird durch die im Kennzeichenteil der Ansprüche 1, 4 bzw. 5 angegebenen Merkmale gelöst. In den Unteransprüchen 2-3 sowie 7-10 sind vorteilhafte Weiterentwicklungen der Gegenstände derjenigen Ansprüche, auf die sie jeweils rückbezogen sind, angegeben. Dem Anspruch 6 ist ein Verfahren zur Herstellung einer erfindungsgemäßen Koaxialkabelanordnung nach Anspruch 4 oder 5 zu entnehmen. Der Anspruch 11 beinhaltet eine Kombination der erfindungsgemäßen Koaxialkabelanordnung mit einem dafür geeigneten Dilatationskatheter. Die Ansprüche 12 und 13 beschreiben schließlich ein Verfahren zur Anwendung der erfindungsgemäßen Koaxialkabelanordnung in Zusammenwirken mit einem Dilatationskatheter.

Dadurch, daß beispielsweise inhärente Wirk,-Flecht- oder Wickellücken im Schirm der erfindungsgemäßen Koaxialkabelanordnung mit Lötzinn oder elektrisch leitfähigem Kunststoff verfüllt werden, wird in einem proximal zum Anwender gelegenen Kabelbereich sowohl eine beim Einführen der Anordnung in einen Dilatationskatheter vorteilhafte größere mechanische Steifigkeit als auch eine Verminderung unerwünschter parasitärer Hochfrequenzabstrahlung im proximalen Kabelbereich erreicht.

Die Versteifung eines proximalen Kabelbereiches kann auch dadurch erzielt werden, daß der um den Innenleiter angeordnete Isolator bzw. der den Schirm umgebende Mantel im proximalen Bereich aus einem steiferen Kunststoff hergestellt wird. In einem distalen Bereich weist der Isolator bzw. Mantel eine demgegenüber geringere Steifigkeit auf, damit die Anordnung beispielsweise durch enge Koronararterien leicht und ohne Verletzungsgefahr geschoben werden kann. Die Herstellung einer solchen Ausführungsform der erfindungsgemäßen Antennenanordnung kann besonders vorteilhaft durch einen Extrudierprozeß erfolgen, bei dem die Extrudiermasse umgeschaltet wird.

Bei einer Dilatationsbehandlung mit einem Dilatationskatheter erweist es sich als vorteilhaft, einen separaten Lumen vorzusehen, durch den eine Kühlflüssigkeit hindurchgeleitet wird, um parasitäre Wärme, die durch parasitäre Hochfrequenzabstrahlung der Koaxialkabelanordnung im proximalen Bereich anfällt, abführen zu können.

Anhand der Zeichnung werden im folgenden mehrere Ausführungsbeispiele der Erfindung erläutert und deren Vorteile dargestellt. Es zeigen:
- **Fig. 1:**: einen Querschnitt durch ein Ausführungsbeispiel einer erfindungsgemäßen Antennenanordnung.
- **Fig. 2:**: einen Querschnitt durch ein weiteres Ausführungsbeispiel einer erfindungsgemäßen Antennenanordnung mit einem Antennenformkörper.
- **Fig. 3:**: ein Diagramm, das den Verlauf der Kabeldämpfung einer herkömmlichen Koaxialkabelanordnung gegenüber der Frequenz der Hochfrequenzenergie veranschaulicht.
- **Fig. 4:**: ein Diagramm, das den Verlauf der Kabeldämpfung einer erfindungsgemäßen Koaxialkabelanordnung gegenüber der Frequenz der Hochfrequenzenergie veranschaulicht.
- **Fig. 5:**: eine Außenansicht eines Dilatationskatheters mit einer erfindungsgemäßen Antennenanordnung.
- **Fig. 6:**: einen Querschnitt A---A durch den in **Fig. 5** dargestellten Dilatationskatheter.

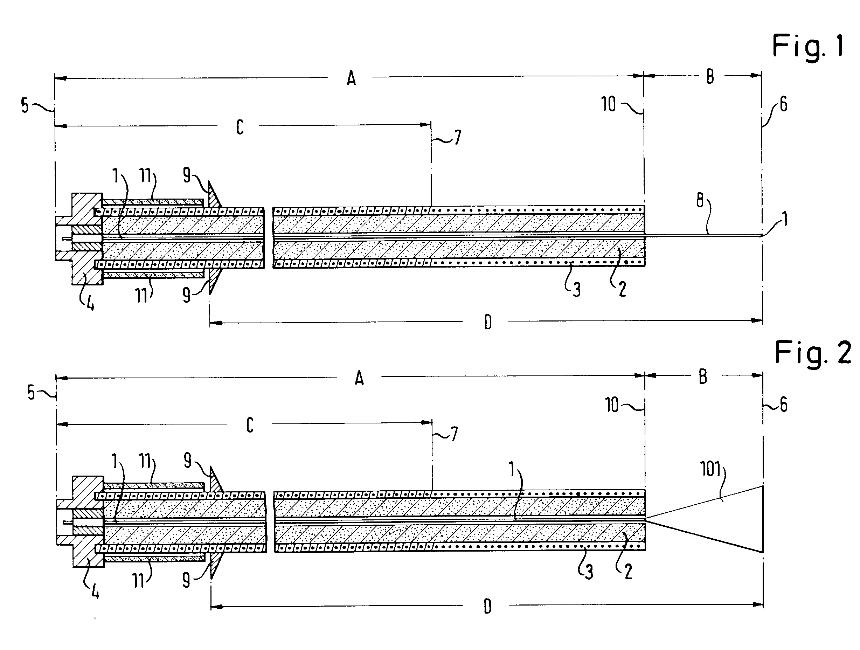

In **Fig. 1** werden die Grundideeen der erfindungsgemäßen Antennenanordnung an einem Ausführungsbeispiel veranschaulicht. Ein durchgehender Innenleiter 1 verläuft von einem proximalen Ende 5, das beispielsweise in Gestalt einer Steckverbindung 4 ausgeführt sein kann, die geeignet ist, die Koaxialkabelanordnung mit einem im Mikrowellenbereich arbeitenden Hochfrequenzgenerator (nicht dargestellt) lösbar zu verbinden, bis zu einem distalen Ende 6. In einem ersten Abschnitt A der Koaxialkabelanordnung, der am proximalen Ende 5 beginnt und vorteilhaft eine Länge von einigen Dezimetern bis zu mehr als einem Meter aufweist, weist die Koaxialkabelanordnung einen zylindrisch um den durchgehenden Innenleiter 1 angeordneten Isolator 2 sowie eine auf den Isolator 2 aufgebrachte zusammenhängende Abschirmeinrichtung 3 auf, die beispielsweise aus dünnem Metalldraht oder aus dünnem metallischen Flachbandleitern gewirkt, geflochten oder gewickelt ist. In einem zweiten Abschnitt B liegt der Innenleiter frei und kann im Zusammenwirken mit der Abschirmeinrichtung 3 als asymmetrischer Stabdipol die Funktion einer Sendeantenne 8 für Hochfrequenzenergie im Mikrowellenbereich erfüllen. Die Länge des zweiten Abschnittes B beträgt beispielsweise einige Zentimeter. Um der Koaxialkabelanordnung im proximalen Bereich eine Mindeststeifigkeit zu verleihen die für das Einführen und Vorschieben der Koaxialkabelanordnung in einem Führungslumen eines Dilatationskatheters erforderlich ist, ist ein dritter Abschnitt C der Koaxialkabelanordnung festgelegt, der am proximalen Ende 5 beginnt und auf jeden Fall kürzer als der erste Abschnitt A ist.

Dieser dritte Abschnitt C der erfindungsgemäßen Koaxialkabelanordnung ist beispielsweise durch eine Schwallbadbehandlung so behandelt worden, daß kleine, der gewirkten bzw. gewickelten Abschirmeinrichtung 3 inhärente Lücken durch eine versteifende leitfähige Masse, in diesem Beispiel Lötzinn, verfüllt sind. Die Erfindung ist jedoch nicht auf die Verwendung von Lötzinn als Verfüllmasse beschränkt, vielmehr sind alle Verfüllmassen verwendbar, die genügend elektrische Leitfähigkeit und genügende Versteifungswirkung aufweisen. Beispielsweise sind auch elektrisch leitfähige Kunststoffe geeignet.

Für Anwendungen, bei denen eine höhere Flexibilität am distalen Ende der Koaxialkabelanordnung nicht benötigt wird, weil keine Körperhohlräume mit engen Biegungen durchlaufen werden, und bei denen es nur auf die Hochfrequenzeigenschaften ankommt, kann es vorteilhaft sein, den gesamten Abschnitt A mit Verfüllmasse zu versehen.

Der Abschnitt zwischen dem distalen Ende 7 des dritten Abschnittes C und dem proximalen Ende 10 des zweiten Abschnittes B wird nicht versteift, damit die Koaxialkabelanordnung in einem Dilatationskatheter beispielsweise in die Koronararterien an engen Biegungen und dergleichen leicht und ohne Verletzungsgefahr eingeführt werden kann.

Bei einer bevorzugten Ausführungsform weist die erfindungsgemäße Koaxialkabelanordnung im Bereich des proximalen Endes 5 einen Einführungsanschlag 9 auf, der in einer vorbestimmten Entfernung D zum distalen Ende 6 angebracht ist, um dem Anwender anzuzeigen, wie weit die Koaxialkabelanordnung in einen Katheter (nicht dargestellt) eingeführt werden darf, und um ein weitergehendes Einführen zu verhindern. Der Bereich der Koaxialkabelanordnung vom proximalen Ende 5 bis zum Einführungsanschlag 9 wird vorteilhaft mit einem isolierenden äußeren Mantel 11 umhüllt. Der Anschlag kann auch durch die Stirnfläche des an der Stelle des Anschlages aufhörenden Mantels 11 gebildet sein.

**Fig 2** zeigt ein weiteres Ausführungsbeispiel einer erfindungsgemäßen Antennenanordnung mit einem metallischen, elektrisch leitfähigen Antennenformkörper 101, der zur Verbesserung der Hochfrequenzeigenschaften am zweiten Abschnitt B der Koaxialkabelanordnung angebracht ist. Bevorzugt wird eine Kegelform, wobei die stumpfe Basis des Kegels das äußerste distale Ende 6 der Koaxialkabelanordnung bildet. Durch den Antennenformkörper 101 wird eine günstigere Anpassung des Wellenwiderstandes der Koaxialkabelanordnung an den Wellenwiderstand des im Bereich des Katheterballons liegenden Gewebes erreicht. Hinsichtlich der übrigen Gestaltungsmerkmale entspricht dieses Ausführungsbeispiel demjenigen Ausführungsbeispiel, das vorstehend mit Bezug auf **Fig. 1** erläutert ist. Gleiche Bestandteile sind mit gleichen Bezugszeichen gekennzeichnet.

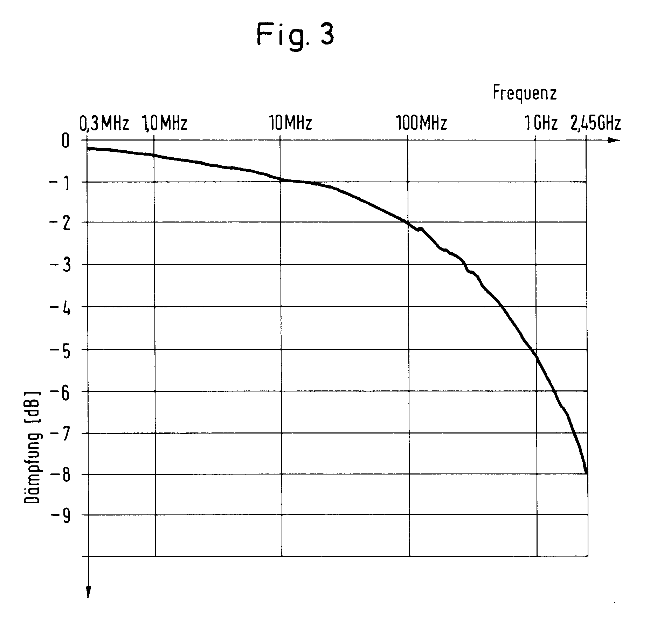

**Fig. 3** ist ein Diagramm, das den Verlauf der Kabeldämpfung eines 3 m langen nicht erfindungsgemäßen Vergleichs-Koaxialkabels auf der Ordinate (nach unten abgetragen) gegenüber der Frequenz der Hochfrequenzenergie auf der Abszisse in einem Frequenzbereich von 0,3 MHz bis 2,5 GHz veranschaulicht, wobei das Vergleichs-Koaxialkabel dem für die erfindungsgemäße Koaxialkabelanordnung verwendeten gleicht, außer daß inhärente Wirk-, Flecht- bzw. Wickellücken in der Abschirmeinrichtung 3 nicht durch eine leitfähige Verfüllmasse verfüllt sind.

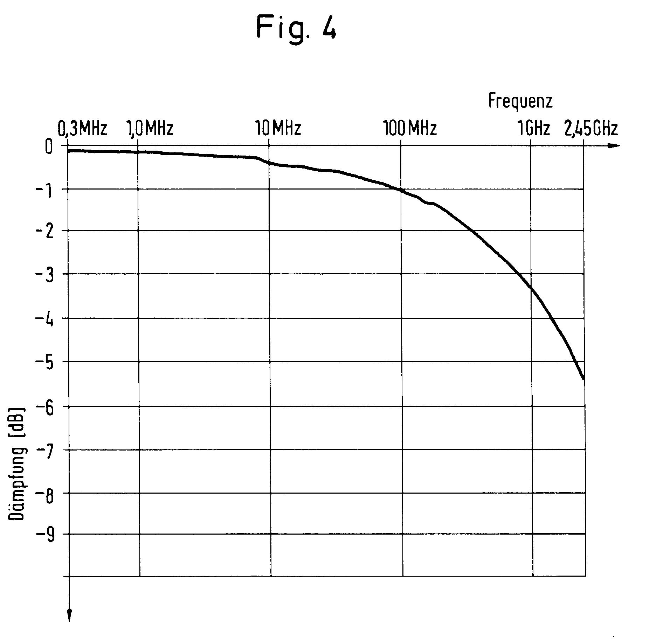

**Fig. 4** ist ein Diagramm, das den Verlauf der Kabeldämpfung eines 3 m langen Stückes des für die erfindungsgemäße Koaxialkabelanordnung verwendeten Koaxialkabels auf der Ordinate (nach unten abgetragen) gegenüber der Frequenz der Hochfrequenzenergie auf der Abszisse in einem Frequenzbereich von 0,3 MHz bis 2,5 GHz veranschaulicht, wobei kleine Wirk- bzw. Wickellücken in der Abschirmeinrichtung 3 durch ein Schwallbad mit Lötzinn verfüllt sind.

Durch Vergleich der **Fig. 3** mit der **Fig. 4** wird ersichtlich, daß die Kabeldämpfung bei dem für die erfindungsgemäße Koaxialkabelanordnung verwendeten Koaxialkabel in wesentlich geringerem Maße ansteigt als bei dem Vergleichskabel in **Fig. 3.** Wie aus den Figuren ersichtlich, beträgt die Kabeldämpfung bei einer Frequenz von 2,45 GHz beim Vergleichskabel ca. -2,7 dB/m, während bei der erfindungsgemäßen Lösung ca. -1,83 dB/m erzielt werden. Dies bedeutet, daß die parasitäre Abstrahlung von Hochfrequenzenergie außerhalb des eigentlichen Antennenbereiches 8, 101 durch das Verfüllen der kleinen Lücken vermindert ist, so daß daher auch die durch die parasitär abgestrahlte Hochfrequenzenergie in den Blutgefäßen, durch die der Katheter geführt werden muß, um an den Ort der Dilatationsbehandlung zu gelangen, freigesetzte parasitäre Wärme vermindert wird. Diese Eigenschaft stellt einen signifikanten Vorteil gegenüber dem Stand der Technik dar, der beispielsweise zu einer Verkürzung der Dilatationsbehandlung genutzt werden kann, indem die Leistung der in die Koaxialkabelanordnung eingespeisten Hochfrequenzenergie erhöht wird. Trotz dieser erhöhten Energieeinspeisung ist somit keine Schädigung der Blutgefäße, durch die der Katheter hindurchgeführt wird, zu befürchten.

Die vorliegende Erfindung ist nicht auf die vorstehend beschriebene Behandlung der Abschirmeinrichtung 3 mit einer Verfüllmasse beschränkt.

Die geforderten mechanischen Eigenschaften der Koaxialkabelanordnung, nämlich eine höhere Steifigkeit im proximalen Bereich und eine demgegenüber geringere Steifigkeit im distalen Bereich, sind auch dann gegeben, wenn für den Isolator 2 in den entsprechenden Bereichen jeweils unterschiedliche Isoliermaterialien mit unterschliedlichen Steifigkeitseigenschaften eingesetzt werden. Eine derartige Koaxialkabelanordnung weist einen dritten Abschnitt C auf, der am proximalen Ende 5 der Antennenanordnung beginnt und der kürzer ist als der erste Abschnitt A der Koaxialkabelanordnung, wobei in dem dritten Abschnitt C der Isolator 2 aus einem ersten Isolationsmaterial besteht. Der Isolator 2 besteht im Bereich zwischen dem distalen Ende 7 des dritten Abschnittes C und dem proximalen Ende 10 des zweiten Abschnittes B aus einem zweiten Isolationsmaterial, wobei das erste Isolationsmaterial eine größere mechanische Steifigkeit als das zweite Isolationsmaterial aufweist. Damit im Grenzbereich zwischen dem ersten und dem zweiten Isolationsmaterial keine unerwünschte Reflexion von Hochfrequenzenergie erfolgt, weisen das erste Isolationsmaterial und das zweite Isolationsmaterial vorzugsweise einen praktisch gleichen Wert für ihre jeweilige Dielektrizitätskonstante auf.

Die erfindungsgemäße Antennenanordnung ist nicht auf eine Anwendung in Blutgefäßen beschränkt; vielmehr ist sie auch für Anwendungen in anderen Körperhohlräumen wie Darm, Vagina etc. geeignet. Insbesondere in solchen Körperhohlräumen, die das Einführen eines Katheters größeren Durchmessers erlauben, kann auf die Abschirmeinrichtung 3 durchgehend bis zum proximalen Ende 10 des zweiten Abschnittes B ein dielektrischer Mantel beispielsweise aus Kunststoff aufgebracht werden. In diesem Fall kann die Steifigkeit der Koaxialkabelanordnung dadurch verändert werden, daß im proximalen Bereich ein Mantel aus einem Umhüllungsmaterial aufgebracht wird, das eine größere Steifigkeit als das Umhüllungsmaterial aufweist, das für den distalen Bereich verwendet wird. Diese Vorgehensweise hat den Vorteil, daß keine Probleme durch reflektierte Hochfrequenzenergie infolge etwa unterschiedlicher Dielektrizitätskonstanten des Isolators 2 entstehen.

Da sowohl die Kabelisolation als auch der Mantel zweckmäßig durch Extrudieren auf den durchgehenden Innenleiter 1 bzw. auf die Abschirmeinrichtung 3 aufgebracht werden, kann eine Koaxialkabelanordnung mit gepaarten Isolierstoffmaterialien bzw. mit gepaarten Umhüllungsmaterialien besonders einfach und kostengünstig hergestellt werden, wenn eine Extrudiervorrichtung verwendet wird, die einen Wechsel der Extrudiermasse ermöglicht, wobei während des Extrudierens der Isolation 2 bzw. des isolierenden Mantels 11 im Bereich des dritten Abschnittes C das erste (steifere) Isoliermaterial bzw. das erste (steifere) Umhüllungsmaterial als Extrudiermasse und während des Extrudierens der Isolation 2 bzw. des dielektrischen Mantels 11 in dem Teil des ersten Abschnittes A, der nicht zum dritten Abschnitt C gehört, das zweite (weniger steife) Isoliermaterial bzw. das zweite (weniger steife) Umhüllungsmaterial als Extrudiermasse eingesetzt wird.

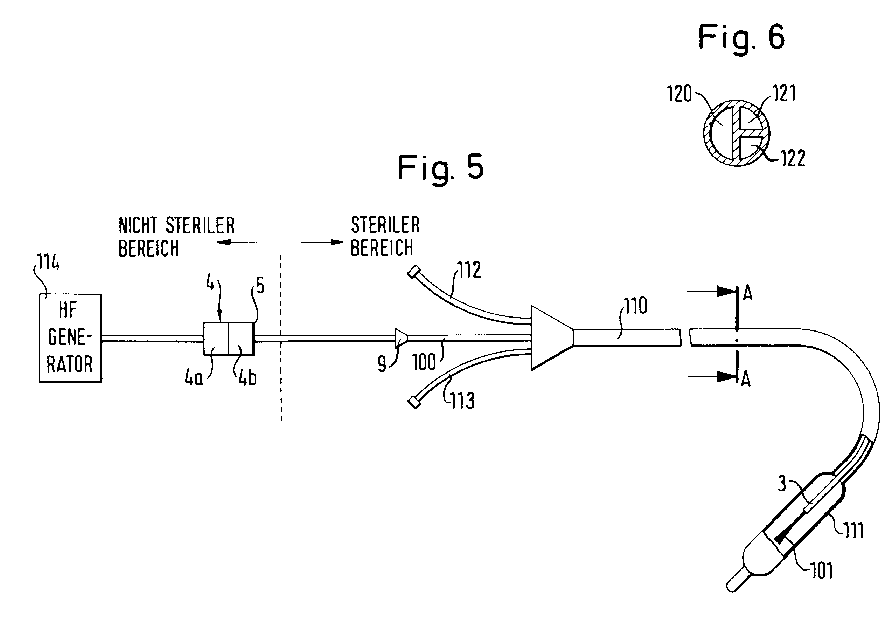

**Fig. 5** stellt eine Außenansicht eines Dilatationskatheters 110 mit einer darin eingesetzten erfindungsgemäßen Koaxialkabelanordnung 100 mit daran angebrachter Antennenanordnung dar. Ein Hochfrequenzgenerator 114 erzeugt Hochfrequenzenergie einer Frequenz von beispielsweise 2,45 GHz, die über eine Steckverbindung 4a, 4b in die Koaxialkabelanordnung 100 eingespeist und mittels des aus dem Antennenformstück 101 und der Abschirmeinrichtung 3 gebildeten asymmetrischen Dipols abgestrahlt wird. Das Antennenformstück 101 ist im distalen Ende des Dilatationskatheters 110 angeordnet, das als Ballon 111 ausgebildet ist. Neben einem ersten Lumen 120, in das die Koaxialkabelanordnung 100 bis zu dem durch den Einführanschlag 9 markierten Punkt eingeführt ist, weist der Katheter 110 noch mindestens zwei weitere Lumen 121,122 auf, wie in **Fig. 6** gezeigt ist. In einen zweiten Lumen 121 ist eine Leitung 112 eingeführt, mittels derer eine Kühlflüssigkeit, beispielsweise eine Salzlösung, durch den Katheter hindurchgeleitet werden kann. Durch diese Kühlflüssigkeit kann die durch parasitäre Hochfrequenzstrahlung der Koaxialkabelanordnung 100 entstehende parasitäre Wärme von der Gefäßwand abgehalten und in unbedenklicher Weise weggeführt werden. In einen dritten Lumen 122 ist ein Führungsdraht 113 eingeführt. Der sterile Bereich des gezeigten Dilatationskatheters beginnt am proximalen Ende 5 der Koaxialkabelanordnung 100 kurz nach der Steckverbindung 4 und erstreckt sich bis zum äußersten distalen Ende des Ballons 111.

**Fig. 6** zeigt einen Querschnitt A---A durch den in **Fig. 5** dargestellten Dilatationskatheter 110 mit dem ersten Lumen 120 zur Aufnahme der Koaxialkabelanordnung 100, mit dem zweiten Lumen 121 zur Aufnahme der Kühlflüssigkeit und mit dem dritten Lumen 122 zur Aufnahme des Führungsdrahtes 113. Wird nur eine geringe Menge Kühlflüssigkeit eingesetzt, so kann sie durch eine Austrittsöffnung (nicht dargestellt) in den Blutkreislauf abgegeben werden. In anderen Ausführungsformen des Dilatationskatheters 110 kann ein weiteres Lumen (nicht dargestellt) vorgesehen sein, durch das die Kühllösung zum Kathetereingang zurückgeführt wird.

## Patentansprüche

1. Antennenanordnung mit Zuleitung in Form einer hochfrequenztauglichen Koaxialkabelanordnung, insbesondere zur medizinischen Wärmeapplikation in Körperhohlräumen, mit einem bezüglich eines einen Eingriff ausführenden Anwenders distalen Ende (6) und einem bezüglich des Anwenders proximalen Ende (5), aufweisend:
(a) einen durchgehenden Innenleiter (1),
(b) einen zylindrisch um den Innenleiter (1) angeordneten Isolator (2),
(c) eine in einem am proximalen Ende (5) der Koaxialkabelanordnung beginnenden ersten Abschnitt (A) der Koaxialkabelanordnung auf den Isolator (2) aufgebrachte Abschirmeinrichtung (3), die aus Metalldraht oder aus Metallfolie gewirkt bzw. gewickelt ist,
(d) einen am distalen Ende (10) des ersten Abschnittes (A) der Koaxialkabelanordnung beginnenden und am distalen Ende (6) der Koaxialkabelanordnung endenden zweiten, als Antenne (8) dienenden Abschnitt (B) der Koaxialkabelanordnung, in dem keine Abschirmeinrichtung (3) um den durchgehenden Innenleiter (1) herum angeordnet ist,
**dadurch gekennzeichnet, daß**
(e) die Koaxialkabelanordnung einen dritten Abschnitt (C) aufweist, der am proximalen Ende (5) der Koaxialkabelanordnung beginnt und der kürzer ist als der erste Abschnitt (A) der Koaxialkabelanordnung, wobei in dem dritten Abschnitt (C) Wirk- bzw. Wickellücken in der Abschirmeinrichtung (3) durch eine versteifende leitfähige Masse verfüllt sind.

2. Antennenanordnung nach Anspruch 1, dadurch gekennzeichnet, daß die leitfähige Verfüllmasse Lötzinn ist.

3. Antennenanordnung nach Anspruch 1, dadurch gekennzeichnet, daß die leitfähige Verfüllmasse elektrisch leitfähiger Kunststoff ist.

4. Antennenanordnung mit Zuleitung in Form einer hochfrequenztauglichen Koaxialkabelanordnung, insbesondere zur medizinischen Wärmeapplikation in Körperhohlräumen, mit einem bezüglich eines einen Eingriff ausführenden Anwenders distalen Ende (6) und einem bezüglich des Anwenders proximalen Ende (5), aufweisend:
(a) einen durchgehenden Innenleiter (1),
(b) einen zylindrisch um den Innenleiter (1) angeordneten Isolator (2),
(c) eine in einem am proximalen Ende (5) der Koaxialkabelanordnung beginnenden ersten Abschnitt (A) der Koaxialkabelanordnung auf den Isolator (2) aufgebrachte Abschirmeinrichtung (3), die aus Metalldraht oder aus Metallfolie gewirkt bzw. gewickelt ist,
(d) einen am distalen Ende (10) des ersten Abschnittes (A) der Koaxialkabelanordnung beginnenden und am distalen Ende (6) der Koaxialkabelanordnung endenden zweiten, als Antenne (8) dienenden Abschnitt (B) der Koaxialkabelanordnung, in dem keine Abschirmeinrichtung (3) um den durchgehenden Innenleiter (1) herum angeordnet ist,
**dadurch gekennzeichnet,**
(e) daß die Koaxialkabelanordnung einen dritten Abschnitt (C) aufweist, der am proximalen Ende (5) der Koaxialkabelanordnung beginnt und der kürzer ist als der erste Abschnitt (A) der Koaxialkabelanordnung, wobei in dem dritten Abschnitt (C) der Isolator (2) aus einem ersten Isolationsmaterial besteht,
(f) daß der Isolator (2) im Bereich zwischen dem distalen Ende (7) des dritten Abschnittes (C) und dem proximalen Ende (10) des zweiten Abschnittes (B) aus einem zweiten Isolationsmaterial besteht, und
(g) daß das erste Isolationsmaterial eine größere mechanische Steifigkeit aufweist als das zweite Isolationsmaterial.

5. Antennenanordnung mit Zuleitung in Form einer hochfrequenztauglichen Koaxialkabelanordnung, insbesondere zur medizinischen Wärmeapplikation in Körperhohlräumen, mit einem bezüglich eines einen Eingriff ausführenden Anwenders distalen Ende (6) und einem bezüglich des Anwenders proximalen Ende (5), aufweisend:
(a) einen durchgehenden Innenleiter (1),
(b) einen zylindrisch um den Innenleiter (1) angeordneten Isolator (2),
(c) eine in einem am proximalen Ende (5) der Koaxialkabelanordnung beginnenden ersten Abschnitt (A) der Koaxialkabelanordnung auf den Isolator (2) aufgebrachte Abschirmeinrichtung (3), die aus Metalldraht oder aus Metallfolie gewirkt bzw. gewickelt ist,
(d) einen am distalen Ende (10) des ersten Abschnittes (A) der Koaxialkabelanordnung beginnenden und am distalen Ende (6) der Koaxialkabelanordnung endenden zweiten, als Antenne (8) dienenden Abschnitt (B) der Koaxialkabelanordnung, in dem keine Abschirmeinrichtung (3) um den durchgehenden Innenleiter (1) herum angeordnet ist,
**dadurch gekennzeichnet,**
(e) daß die Koaxialkabelanordnung einen dritten Abschnitt (C) aufweist, der am proximalen Ende (5) der Koaxialkabelanordnung beginnt und der kürzer ist als der erste Abschnitt (A) der Koaxialkabelanordnung, wobei in dem dritten Abschnitt (C) die Abschirmeinrichtung (3) von einem dielektrischen Mantel (11) umhüllt ist, der aus einem ersten Umhüllungsmaterial besteht,
(f) daß die Abschirmeinrichtung (3) im Bereich zwischen dem distalen Ende (7) des dritten Abschnittes (C) und dem proximalen Ende (10) des zweiten Abschnittes (B) von einem dielektrischen Mantel (11) umhüllt ist, der aus einem zweiten Umhüllungsmaterial besteht, und
(g) daß das erste Umhüllungsmaterial eine größere mechanische Steifigkeit aufweist als das zweite Umhüllungsmaterial.

6. Verfahren zur Herstellung einer Koaxialkabelanordnung nach Anspruch 4 oder 5, wobei der Isolator (2) bzw. der dielektrische Mantel (11) durch Extrudieren eines thermoplastischen Isolierstoffes bzw. Umhüllungsmaterials auf den durchgehenden Innenleiter (1) bzw. auf die Abschirmeinrichtung (3) aufgebracht wird,
**dadurch gekennzeichnet,**
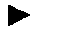 daß während des Extrudierens der Isolation (2) bzw. des dielektrischen Mantels (11) im Bereich des dritten Abschnittes (C) das erste Isoliermaterial bzw. das erste Umhüllungsmaterial als Extrudiermasse eingesetzt wird,
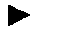 daß während des Extrudierens der Isolation (2) bzw. des dielektrischen Mantels (11) in dem Teil des ersten Abschnittes (A), der nicht zum dritten Abschnitt (C) gehört, das zweite Isoliermaterial bzw. das zweite Umhüllungsmaterial als Extrudiermasse eingesetzt wird.

7. Antennenanordnung nach Anspruch 4, dadurch gekennzeichnet, daß das erste Isolationsmaterial praktisch den gleichen Wert der Dielektrizitätskonstanten aufweist wie das zweite Isolationsmaterial.

8. Antennenanordnung nach einem der Ansprüche 1-5 oder 7, dadurch gekennzeichnet, daß die Koaxialkabelanordnung einen in einer vorbestimmten Entfernung (D) vom distalen Ende (6) auf der Abschirmeinrichtung angeordneten Einführungsanschlag (9) aufweist.

9. Antennenanordnung nach einem der Ansprüche 1-5 oder 7-8, dadurch gekennzeichnet, daß die Koaxialkabelanordnung am distalen Ende (6) einen am Innenleiter (1) befestigten Antennenformkörper (101) aufweist.

10. Antennenanordnung nach Anspruch 9, dadurch gekennzeichnet, daß der Antennenformkörper (101) Kegelgestalt aufweist, wobei die stumpfe Basis des Kegels das distale Ende (6) der Koaxialkabelanordnung bildet.

11. Dilatationskatheter mit mindestens einem Lumen,
**dadurch gekennzeichnet,**
daß in dem Lumen eine Antennenanordnung gemäß einem der Ansprüche 1-5 oder 7-10 angeordnet ist.

12. Dilatationsvorrichtung mit einem mehrlumigen Dilatationskatheter,
**dadurch gekennzeichnet,**
daß in eines der Lumen eine Antennenanordnung gemäß einem der Ansprüche 1-5 oder 7-10 einführbar ist und daß ein zweites der Lumen an eine Kühlflüssigkeitsquelle anschließbar ist.

13. Dilatationsvorrichtung nach Anspruch 12, dadurch gekennzeichnet, daß das zweite Lumen an eine Salzlösungsquelle anschließbar ist.
